# EUROPÄISCHE PATENTSCHRIFT

(11) **EP 4 054 499 B1**
(45) Veröffentlichungstag und Bekanntmachung des Hinweises auf die Patenterteilung: **30.04.2025**
(21) Anmeldenummer: 19801280.9
(22) Anmeldetag: 06.11.2019
(51) Int. Cl.: B32B 3/26, B32B 3/28, B32B 7/05, A61F 13/49, B32B 5/02, B32B 27/12, B32B 27/08, B32B 27/30, B32B 27/32

(54) **VERFAHREN ZUR HERSTELLUNG EINES ELASTISCHEN LAMINATES SOWIE NACH DEM VERFAHREN ERHÄLTLICHES LAMINAT**
METHOD FOR PRODUCING AN ELASTIC LAMINATE, AND LAMINATE OBTAINABLE ACCORDING TO SAID METHOD
PROCÉDÉ DE FABRICATION D'UN STRATIFIÉ ÉLASTIQUE ET STRATIFIÉ ÉLASTIQUE CORRESPONDANT

(43) Veröffentlichungstag der Anmeldung: 14.09.2022
(73) Patentinhaber: Nitto Advanced Film Gronau GmbH, 48599 Gronau (DE)
(72) Erfinder: TRINKAUS, Jan Michael, 53881 Euskirchen (DE)
(74) Vertreter: Andrejewski - Honke Patent- und Rechtsanwälte Partnerschaft mbB
(86) Internationale Anmeldenummer: PCT/EP2019/080423
(87) Internationale Veröffentlichungsnummer: WO 2021/089141

(56) Entgegenhaltungen:
- US-A1- 2005 101 216
- US-A1- 2016 058 624

## Beschreibung

Die Erfindung betrifft ein Verfahren zur Herstellung eines elastischen Laminates mit zumindest einer ersten Decklage und einer elastischen Folie. Gegenstand der Erfindung ist des Weiteren ein Laminat, welches durch das Verfahren erhältlich ist.

Das elastische Laminat wird besonders bevorzugt als elastischer Abschnitt eines Wegwerf-Hygieneartikels eingesetzt. Das elastische Laminat ist dabei insbesondere als elastisches Verschlussband einer herkömmlichen Windel oder auch als elastischer Seitenabschnitt einer Höschenwindel vorgesehen, die in der Praxis auch Training-Pants genannt werden. Neben Windeln für Babys sind entsprechende elastische Materialien auch bei Inkontinenz-Artikeln für ältere Kinder und Erwachsene vorgesehen.

Im Hinblick auf die beschriebenen Anwendungsfälle sind verschiedene Randbedingungen zu beachten. Einerseits werden bei Wegwerf-Artikeln möglichst geringe Kosten und eine möglichst effiziente Materialausnutzung angestrebt. Auch die Herstellung und Verarbeitung soll möglichst einfach und kostengünstig sein.

Darüber hinaus sind die beschriebenen Einsatzzwecke jedoch technisch anspruchsvoll, weil eine hohe Zuverlässigkeit sowie ein guter Tragekomfort notwendig sind. Insbesondere müssen die beschriebenen Hygieneartikel auch bei Bewegungen des Benutzers eine gute Passform aufweisen, damit aufgenommene Ausscheidungen sicher zurückgehalten werden. Darüber hinaus sollen die beschriebenen Hygieneartikel auch nicht als unangenehm empfunden werden oder sogar zu einer Beeinträchtigung des Benutzers führen.

Die bekannten elastischen Laminate weisen zu diesem Zweck in der Regel zumindest eine Decklage aus Nonwoven auf, welches eine weiche, angenehme und textilartige Oberfläche bildet und kostengünstig hergestellt werden kann. Bei einer solchen üblicherweise nicht elastischen Decklage werden dann die gewünschten elastischen Rückstelleigenschaften für einen sicheren Sitz durch die elastische Folie erreicht. Bei der Decklage ist lediglich sicherzustellen, dass das gebildete Laminat in einem ausreichenden Maße dehnbar ist.

Um diese Eigenschaften des Laminates zu erreichen, sind aus der Praxis verschiedene Ansätze bekannt. Beispielsweise können eine vorproduzierte elastische Folie und eine vorproduzierte Decklage aus Nonwoven weitgehend spannungsfrei unter Einsatz von Druck und Temperatur, Klebstoff oder durch ein Ultraschallverschweißen miteinander verbunden werden, wobei vorzugsweise ein Muster aus verbundenen und unverbundenen Bereichen vorgesehen ist. Wenn das Nonwoven dann nur eine geringe Festigkeit aufweist, kann das so gebildete Laminat unmittelbar für die zuvor beschriebenen Zwecke eingesetzt werden.

Häufig ist jedoch noch während des Herstellungsprozesses eine Aktivierung des gebildeten Laminates vorgesehen, wozu das Laminat in einer gewünschten Dehnrichtung, beispielsweise quer zu einer Produktionsrichtung, gedehnt wird. Hierzu kann das Laminat beispielsweise einer Dehneinrichtung mit Ringrollen zugeführt werden. Durch den wellenförmigen oder zick-zack-förmigen Verlauf des Walzenspaltes von ineinandergreifenden Ringrollen wird das eingeführte Material in einer Querrichtung verlängert, wodurch die dehnbare, jedoch nicht elastische Decklage aus Nonwoven überdehnt, bleibend verformt und gegebenenfalls auch teilweise zerstört wird. Bei dem Verlassen des Walzenspaltes ergibt sich eine elastische Rückstellung aufgrund der elastischen Folien, wobei dann das Laminat bis zu dem Grad der vorherigen Verstreckung nachfolgend leicht dehnbar ist. Durch die plastische Verformung und/oder teilweise Zerstörung der Decklage werden dann die elastischen Eigenschaften weitgehend von der elastischen Folie bestimmt.

Der beschriebene Ansatz ist beispielsweise in der EP 0 573 586 B1 beschrieben und in der Praxis weit verbreitet.

Da die elastischen Folien und die zumindest eine Decklage bei der Laminierung weitgehend ungedehnt sind und nachfolgend in der Regel auch noch eine Aktivierung vorgesehen ist, werden entsprechende Materialien auch als aktivierbares Laminat, Zero-Strain-Laminat oder latent-elastisches Laminat bezeichnet.

Auch wenn die beschriebenen Laminate sich bewährt haben, sind spezifische Nachteile zu berücksichtigen. Da bei der Aktivierung das Nonwoven überstreckt und häufig auch zum Teil zerstört ist, kann das Laminat bei der Benutzung auch relativ leicht bis zu einer vollständigen Zerstörung weiter gedehnt werden. Für einen Benutzer ergibt sich keine klar wahrnehmbare Dehngrenze.

Eine Variante eines Zero-Strain-Laminates ist in der EP 2 177 654 B1 beschrieben, wobei direkt auf den elastischen Folien zumindest einseitig eine Schicht aus Nonwoven gebildet wird. Das Nonwoven kann dabei auch so leicht dehnbar sein, dass eine vorherige Aktivierung nicht notwendig ist. Gerade deshalb resultiert aber auch hier keine für einen Benutzer wahrnehmbare Dehngrenze.

Gemäß alternativen Ansätzen wird erreicht, dass eine in ihrer Struktur nicht wesentlich beeinträchtigte Decklage aus Nonwoven nur an einzelnen, insbesondere linienförmigen oder punktförmigen Abschnitten mit der elastischen Folie verbunden ist und dazwischen wellenförmig aufgeworfen ist. Grundsätzlich kann eine solche Struktur auch erreicht werden, wenn bei den zuvor beschriebenen Laminaten beispielsweise mittels Klebstoff nur eine abschnittsweise Verbindung erfolgt und das Nonwoven-Material bei der Aktivierung nicht zerstört wird.

Diese Struktur wird jedoch auch erreicht, wenn die elastische Folie bei der Kaschierung entlang der gewünschten Dehnrichtung gedehnt ist und abschnittsweise mit der Decklage verbunden wird. Nach dem Wegfall der Zugkräfte stellt sich die elastische Folie dann zurück und schiebt somit auch die daran befestigte Decklage wellenförmig zusammen. Es ergibt sich der Vorteil, dass das Nonwoven noch weitgehend seine ursprüngliche Struktur aufweist und lediglich entlang der bevorzugten Dehnrichtung zusammen geschoben wird. Das so gebildete Laminat kann dann nachfolgend bis zu dem Grad der Dehnung bei der Kaschierung sehr leicht gedehnt werden, bis dann die zunächst wellenförmig auf der elastischen Folie aufliegende Decklage gewissermaßen wieder gerade gezogen ist. Je nach Ausgestaltung der Decklage kann auf besonders vorteilhafte Weise eine sehr deutliche Dehngrenze erreicht werden. In dem Kraft-Dehnungsdiagramm des Laminates ergibt sich dann ein deutlich wahrnehmbarer steiler Anstieg der für eine weitere Dehnung erforderlichen Kraft.

Da die elastische Folie bei der Kaschierung gedehnt ist, wird eine solche Verfahrensführung auch als Stretch-Bonding bezeichnet.

Ein Material mit vergleichbaren Eigenschaften kann gebildet werden, wenn gemäß einer weiteren Variante die Decklage vor der Verbindung mit der elastischen Folien entlang der gewünschten Dehnrichtung in eine Wellenform gebracht und dann mit der elastischen Folie verbunden wird. Eine entsprechende Handhabung der Decklage kann anspruchsvoll sein, insbesondere weil die Ränder der entsprechenden Materialbahn hinreichend genau positioniert werden müssen. Als Vorteil ergibt sich jedoch, dass auf die elastische Folie bei der Kaschierung entlang der gewünschten Dehnrichtung keine großen Zugkräfte aufgebracht werden müssen. Da die Decklage für die Kaschierung mit ihrer Wellenform in Falten gelegt wird, wird eine entsprechende Verfahrensführung in der Praxis auch als Neck-Bonding bezeichnet.

Ein solches Neck-Bonding ist aus der EP 0 985 394 B1 bekannt, wobei jedoch dort die Verbindung der Decklage mit einzelnen elastischen Strängen und nicht mit einer Folie beschrieben ist. Aus der Praxis sind jedoch auch Ausgestaltungen bekannt, bei denen mittels Neck-Bonding zumindest eine Decklage aus Nowoven mit einer elastischen Folie kaschiert wird.

Ob mittels ineinander greifenden Ringrollen eine Materialbahn gedehnt oder lediglich in Falten gelegt wird, hängt von verschiedenen Faktoren wie der Form der Ringrollen sowie der wirkenden Reibung ab. Häufig ist es für ein Dehnen auch notwendig, die Materialbahnen an ihren Rändern festzuhalten, wie dies bei dem fertigen Laminat auch gemäß der bereits zuvor genannten EP 0 573 586 B1 vorgesehen ist.

Das Dehnen einer Deckschicht vor seiner Weiterverarbeitung mittels Ringrollen ist beispielsweise aus der WO 2017/184542 A1 bekannt. Bei einer um den Umfang gleichbleibenden Struktur der Ringrollen wird dabei eine durchgehende Wellenform erzeugt. Alternativ kann die Struktur der einzelnen vorstehenden Ringe auch entlang des Umfanges variiert werden, um dann auch entlang der Produktionsrichtung unterschiedliche Strukturen bilden zu können. Entsprechende Maßnahmen sind aus der WO 2017/184542 A1 bekannt.

Vor dem Hintergrund des Standes der Technik liegt der vorliegenden Erfindung die Aufgabe zugrunde, ein Verfahren zur Herstellung eines elastischen Laminates anzugeben, wobei das gebildete Laminat besonders kostengünstig ist und gute Funktionseigenschaften aufweist. Des Weiteren soll ein Laminat angegeben werden, welches durch das Verfahren erhältlich ist.

Gegenstand der Erfindung und Lösung der Aufgabe sind ein Verfahren zur Herstellung eines elastischen Laminates gemäß Patentanspruch 1 sowie ein mit dem Verfahren erhältliches Laminat gemäß Patentanspruch 16.

Die Erfindung betrifft somit ein Verfahren zur Herstellung eines elastischen, insbesondere eines querelastischen Laminates mit zumindest einer ersten Decklage und einer elastischen Folie, wobei die erste Decklage als Materialbahn entlang einer Produktionsrichtung einer ersten Dehneinrichtung zugeführt wird, die erste Decklage nachfolgend mittels der ersten Dehneinrichtung quer zur Produktionsrichtung gedehnt und dabei in Falten gelegt wird und die in Falten gelegte erste Decklage nachfolgend im in Falten gelegten Zustand entweder mit einer für die Bildung der elastischen Folie vorgesehenen Extrusionsbahn derart verbunden wird, dass die Decklage an ihrer der Extrusionsbahn zugewandten Seite lediglich an Abschnitten mit der Extrusionsbahn verbunden wird oder mit einer vorgefertigten elastischen Folie dadurch verbunden wird, dass die elastische Folie an ihrer der ersten Decklage zugewandten Seite zumindest angeschmolzen wird, dass die erste Decklage lediglich an Abschnitten gegen die zumindest angeschmolzene Seite der elastischen Folie gedrückt wird und dass dabei die erste Decklage zumindest teilweise in eine Polymermatrix der elastischen Folie eingebettet wird.

Gemäß einem ersten Aspekt der Erfindung ist also vorgesehen, dass die erste Decklage im Rahmen ihrer Dehnung mittels der ersten Dehneinrichtung auch in Falten gelegt wird, wobei dann die Falten zumindest teilweise und vorzugsweise zumindest weitgehend bis zu der Kaschierung beibehalten werden. Bei der Kaschierung ist also die erste Decklage noch in Falten gelegt, so dass für die vorgesehene elastische Dehnung des gebildeten Laminates durch die Falten ein Materialvorrat bereitgestellt wird.

Gemäß einer bevorzugten Ausgestaltung der Erfindung ist dabei vorgesehen, dass die erste Dehneinrichtung von zwei ineinandergreifenden Ringrollen gebildet ist, wobei dann die erste Materialbahn bis zu ihrer Kaschierung an einer der Ringrollen geführt werden kann. Zunächst kann dadurch erreicht werden, dass die erste Materialbahn bei einer genau vorgegebenen Breite, insbesondere der Ausgangsbreite der Materialbahn auch nach der Dehnung zunächst in ihrer Struktur unverändert bleibt, wobei die Materialbahn dann an den Vorsprüngen der zugeordneten Ringrolle gehalten und geführt ist.

Um die erste Materialbahn bei genau oder zumindest in etwa bei einer vorgegebenen Breite zu halten, wird diese von der ersten Dehneinrichtung zweckmäßigerweise an ihren Rändern festgehalten und insbesondere an ringförmigen Rändern der Ringrollen eingeklemmt. Wenn dann zwischen den Rändern über eine Mittelebene vorstehende, wechselweise ineinander greifende Ringe äquidistant angeordnet sind, ergibt sich zwischen den Rändern eine im Mittel gleichmäßige Dehnung, wobei jedoch durch die Reibung und Abstützung der ersten Materialbahn an den einzelnen Ringen, die auch als Zähne bezeichnet werden, eine Modulation der Dehnung entsprechend dem Abstand der aufeinander folgenden Ringe möglich ist. Insbesondere kann sich dabei ergeben, dass die erste Materialbahn an den Kontaktstellen mit den Ringen weniger und zwischen den Ringen stärker gedehnt wird.

Vor diesem Hintergrund ist die Erfindung jedoch nicht auf Dehnungseinrichtungen beschränkt, bei denen die Ringe äquidistant angeordnet sind. Vielmehr kann auch der Abstand der aufeinander folgenden Ringe variiert werden, um gezielt Bereiche mit unterschiedlicher Dehnbarkeit zu erzeugen und somit die mechanischen Eigenschaften des Laminates noch genauer an konkrete Anforderungen anpassen zu können.

Zusätzlich oder alternativ kann die erste Materialbahn nicht nur an ihren Rändern sondern auch an zumindest einen Zwischenabschnitt festgehalten werden, damit diese dort nicht gedehnt und in Falten gelegt wird. Eine solche Verfahrensführung kann beispielsweise zweckmäßig sein, um das Laminat mehrnutzig weiterverarbeiten zu können, d.h. in Querrichtung zumindest zwei aufeinander folgende Streifen zu bilden. Die Trennung der zumindest zwei aufeinander folgenden Streifen erfolgt dann zweckmäßigerweise an dem Zwischenabschnitt. Da die erste Materialbahn dann an dem Zwischenabschnitt flach und ungedehnt ist, können die so gebildeten Streifen leicht weitererarbeitet und beispielsweise bei der Herstellung eine Hygieneartikel verklebt werden.

Bei einer Justagemöglichkeit einzelner Ringe oder Ringsegmente kann gegebenenfalls auch der Spalt für die Verbindung mit der Extrusionsbahn bzw. der elastischen Folie optimiert werden.

Besonders bevorzugt können dann die erste Materialbahn und die Extrusionsbahn bzw. die elastische Folie in einem Walzenspalt zwischen der entsprechenden Ringrolle und einer Gegenwalze verbunden werden. An den einzelnen Vorsprüngen kann dann die Decklage effektiv und mit einer vergleichsweise großen Kraft gegen die Extrusionsbahn bzw. die elastische Folie gedrückt werden, was zu einer besonders innigen und zuverlässigen Verbindung führt, während zwischen den Vorsprüngen die Decklage von der Extrusionsbahn der elastischen Folie beabstandet bleibt und somit dort keine Verbindung erfolgt.

Gemäß einer bevorzugten Ausgestaltung wird die für die Bildung der elastischen Folie vorgesehene Extrusionsbahn erst unmittelbar vor der Kaschierung inline in dem Verfahren zur Herstellung des elastischen Laminates gebildet. Insbesondere kann dabei ihm Rahmen der Erfindung vorgesehen sein, dass die ein- oder mehrschichtige Extrusionsbahn zumindest an ihrer der ersten Decklage zugewandten Seite noch von dem unmittelbar vorgelagerten Extrusionsprozess schmelzfüssig oder zumindest noch nicht vollständig erstarrt ist. Selbst wenn die entsprechende Oberfläche nach dem Extrusionsprozess zunächst erstarrt, kann dann für die Kaschierung die Oberfläche wieder erwärmt und zumindest angeschmolzen werden, wodurch dann die Kaschierung ohne zusätzlichen Klebstoff und somit ohne die Einbringung weiterer chemischer Komponenten möglich. Bei einer mehrschichtigen Ausgestaltung der Extrusionsbahn können für die unterschiedlichen Schichten auch unterschiedliche Schmelzpunkte vorgesehen sein. So kann es für die Verfahrensführung beispielsweise zweckmäßig sein, wenn bei der Kaschierung eine von der ersten Decklage abgewandte Schicht oder eine Kernschicht der Extrusionsbahn schon vollständig oder weitgehend erstarrt ist und somit der Extrusionsbahn bereits eine gewisse Festigkeit verleiht.

Gemäß einer alternativen Ausgestaltung wird eine vorgefertigte elastische Folie zugeführt und dann vor der Verbindung mit der Decklage an ihrer der Decklage zugewandten Seite zumindest angeschmolzen. Die eigentliche Verbindung erfolgt dann wie zuvor im Zusammenhang mit der Extusionsbahn beschrieben nur durch das zusammendrücken der zu verbindenden Lagen ohne zusätzlichen Klebstoff und somit ohne die Einbringung weiterer chemischer Komponenten. Aufgrund des übereinstimmenden Verbindungsprozesses sind die nachfolgend im Zusammenhang mit der Extrusionsbahn beschriebenen Maßnahmen üblicherweise auch für die Verfahrensführung geeignet, bei der eine vorgefertigte elastische Folie zugeführt wird.

Die im Vergleich zu einem vollflächigen Andruck an den einzelnen Vorsprüngen wirkenden erhöhten Andruckkräfte sind für die Erzielung einer hohen lokalen Verbundhaftung von besonderem Vorteil. Während die unverbundenen, Falten bildenden Bereiche weich und beweglich bleiben, wird durch die erhöhten Andruckkräfte eine besonders innige Verbindung erzeugt. Insbesondere können lokal Andruckkräfte erreicht werden, welche bei einer flächigen Verbindung schwer anzuwenden sind oder das Material schädigen könnten. Im Rahmen der Erfindung kann ein gewisses Zusammendrücken der ersten Decklage lediglich an den verbundenen Abschnitten dagegen hingenommen werden. Hinsichtlich der Andruckkräfte ergibt sich gegenüber einem vollflächigen Andruck ein größerer zulässiger Parameterbereich, der auch als Prozessfenster bezeichnet werden kann. Auch ergibt sich eine größere Auswahlmöglichkeit der Materialien für die erste Decklage. Dabei können im Detail jedoch unterschiedliche Aspekte eine Rolle spielen. So ist es möglich, dass die erste Decklage in einem gewissen Maße in die Extrusionsbahn bzw. die elastische Folie oder zumindest eine zugeordnete Schicht der Extrusionsbahn bzw. der elastischen Folie hineingedrückt wird.

Wenn beispielsweise gemäß einer bevorzugten Ausgestaltung der Erfindung als erste Materialbahn ein Nonwoven zugeführt wird, so können dann die einzelnen Fasern des Nonwovens von dem Polymer der Extrusionsbahn bzw. der zumindest angeschmolzenen elastischen Folie umschlossen werden, sodass dann eine Trennung nur noch durch eine Zerstörung oder zumindest teilweise Zerstörung der ersten Decklage bei einer maximalen Verbundhaftung möglich ist.

Alternativ kann die erste Materialbahn beispielsweise auch von einer Folie, einem Gewebe oder einem Gewirke gebildet sein. Es muss lediglich sichergestellt sein, dass die erste Materialbahn quer zur Produktionsrichtung gedehnt und dabei in Falten gelegt werden kann.

Wenn die erste Materialbahn Öffnungen, Durchbrechungen oder Freiräume aufweist, kann wie zuvor im Zusammenhang mit einem Nonwoven beschrieben eine Verbindung durch eine Art Formschluss erreicht werden, wenn das Polymer der Extrusionsbahn bzw. der zumindest angeschmolzenen elastischen Folie in die Öffnungen. Durchbrechungen oder Freiräume eindringt.

Bei allen möglichen Ausgestaltungen der ersten Materialbahn kann zusätzlich oder alternativ vorgesehen sein, dass Bestandteile der ersten Materialbahn durch bei der Kaschierung mit der Extusionsbahn bzw. der elastischen Folie durch Druck und Temperatur auf- bzw. anschmelzen. Wenn die erste Materialbahn beispielsweise von einer Folie gebildet ist, so kann diese eine niedrigschmelzende Deckschicht aufweisen. Wenn die erste Materialbahn aus Fasern oder Fäden gebildet ist, so können diese auch beispielsweise als Faser-Fadengemisch, durch verschiedene Filamente oder einen Bikomponenten-Aufbau niedrigschmelzende und hochschmelzende Bestandteile aufweisen.

Zusätzlich oder auch alternativ kann auch vorgesehen sein, dass die erste Decklage zumindest teilweise aufgrund der Temperatur der Extrusionsbahn bzw. der zumindest angeschmolzenen elastischen Folie auf- oder zumindest angschmolzen wird, sodass die Materialien miteinander zuverlässig verschweißt werden. Wenn - wie zuvor beschrieben - als erste Materialbahn ein Nonwoven eingesetzt wird, so können auch die das Nonwoven bildenden Fasern diesbezüglich optimiert sein.

Beispielsweise kann ein Nonwoven aus verschiedenen Fasern mit einer unterschiedlichen Schmelz- bzw. Erweichungstemperatur gebildet sein. Das Verfahren kann dann so geführt werden, dass lediglich ein Teil der Fasern auf- oder anschmilzt und eine besonders zuverlässige Verbindung ermöglicht, während ein anderer Teil der Fasern nicht auf- oder angeschmolzen wird und somit die strukturelle Integrität des Nonwovens sicherstellt.

Zusätzlich oder alternativ kann zumindest auch ein Teil der Fasern in Form von Mehrkomponenten-Fasern und insbesondere Bikomponenten-Fasern vorliegen. Bei einer Bikomponenten-Faser kann beispielsweise ein hochschmelzender Kern mit einem niedrigschmelzenden Mantel kombiniert werden. Zweckmäßigerweise ist dann vorgesehen, dass lediglich der niedrigschmelzende Mantel auf- bzw. angeschmolzen wird.

Die zuvor beschriebene Gegenwalze wird auch als Glättwalze bezeichnet, weil die Extrusionsbahn daran geführt und abgestützt ist. Zweckmäßigerweise kann die Gegenwalze temperiert sein, um während der Kaschierung und dem weiteren Transport des gebildeten Laminates gewünschte Eigenschaften zu erzielen. So ist es zweckmäßig, wenn nach der Verbindung der Extrusionsbahn mit der ersten Decklage zumindest ein übermäßiges Fließen des entsprechenden Polymers vermieden wird. Die Glätt- bzw. Gegenwalze wird deshalb in der Praxis häufig auch als Chill-Roll bezeichnet.

Vorzugsweise ist vorgesehen, dass an beiden Seiten der Extrusionsbahn bzw. der elastischen Folie jeweils eine Decklage angeordnet ist, wobei hierzu verschiedene Varianten in Betracht kommen. Beispielsweise wird gegenüberliegend der zuvor beschriebenen ersten Decklage eine zweite Decklage angeordnet, wobei auch die zweite Decklage wie zuvor beschrieben zweckmäßigerweise entlang einer Produktionsrichtung einer zweiten Dehneinrichtung zugeführt wird, mittels der zweiten Dehneinrichtung quer zur Produktionsrichtung gedehnt und dabei in Falten gelegt wird und wobei die zweite Decklage nachfolgend in Falten gelegt mit der Extrusionsbahn verbunden wird.

Dabei ist zu berücksichtigen, dass die vorzugsweise aus einer Cast-Düse austretende Extrusionsbahn nach dem Verlassen des Extrusionsspaltes abkühlt, sodass dann die beiden Decklagen rechtzeitig zugeführt und kaschiert werden müssen, bevor die Polymerschmelze zu stark abgekühlt ist.

Grundsätzlich ist es denkbar, dass für eine Ausgestaltung mit zwei Decklagen eine genau oder im Wesentlichen symmetrische Verfahrensführung erfolgt, wobei dann die Kaschierung der beiden Decklagen in einem Walzenspalt erfolgt, der von zwei gegenüberliegenden Ringrollen gebildet ist. Für eine solche Verfahrensführung müssen jedoch die Zähne der Ringrollen einander genau gegenüber stehen, wobei zwischen den Zähnen die Extrusionsbahn nicht geführt ist. Um die zeitgleiche symmetrische oder im Wesentlichen symmetrische Zuführung und Kaschierung von zwei Decklagen zu ermöglichen, können verschiedene spezifische Anpassungen zweckmäßig sein. Beispielsweise ist es zweckmäßig, wenn die Extrusionsbahn zwar noch weich genug für eine innige Verbindung mit den Decklagen ist, jedoch bereits eine gewisse Schmelzefestigkeit aufweist. Ansonsten könnte die Schmelzebahn zwischen den einander entgegenstehenden Zähnen zu stark ausgedünnt werden. Auch eine später noch weiter beschriebene mehrschichtige Ausgestaltung mit zumindest drei Schichten kann zweckmäßig sein, wobei dann beispielsweise bei der Kaschierung eine Kernschicht aufgrund der bereits erfolgten Abkühlung und/oder ihrer Viskosität auch an den Verbindungsstellen ihre vorherige Dicke im Wesentlichen beibehält. Bei einander gegenüberliegenden Ringrollen können die Ringe auch an ihrem äußeren Umfang eben oder zumindest abgeflacht sein, um auch bei einer leichten Fehlausrichtung dennoch eine zuverlässige Verbindung zu erreichen.

Wenn gemäß den zuvor beschriebenen Varianten der Erfindung anstelle der Extusionsbahn eine vorgefertigte elastischen Folie zugeführt wird, wo kann dann beispielsweise auch bei einer beidseitigen Erwärmung für ein Anschmelzen der Oberflächen der Energieeintrag auch so vorgesehen werden, dass zumindest ein Kern oder eine Kernschicht der elastischen Folie nicht aufschmilzt und so auch den Druckkräften an den Abschnitten standhalten kann.

Gemäß einer bevorzugten Weiterbildung der Erfindung ist vorgesehen, dass die erste Decklage und die zweite Decklage sukzessive mit einem Versatz zueinander zugeführt und mit der Extrusionsbahn bzw. der elastischen Folie verbunden werden. Selbstverständlich wird dann der Abstand der beiden Orte für die Kaschierung mit den beiden Decklagen möglichst gering gehalten.

Um an beiden Seiten der Extrusionsbahn bzw. der elastischen Folie mit einem gewissen Versatz eine Verbindung mittels Thermobondierung zu ermöglichen, kann für die Extrusionsbahn bzw. die elastische Folie auch ein Polymermaterial vorgesehen werden, welches über einen größeren Temperaturbereich verarbeitet werden kann, sodass dann zumindest etwas unterschiedliche Temperaturen bei der Kaschierung der ersten Decklage einerseits sowie der zweiten Decklage andererseits hingenommen werden können.

Auch dabei ist zu beachten, dass die Extrusionsbahn bzw. die elastische Folie mehrschichtig ausgeführt sein können, wobei auch dann hinsichtlich der verschiedenen Schichten ein unterschiedliches thermisches Verhalten zweckmäßig ist. Wenn beispielsweise für die Bildung der elastischen Folie eine Kernschicht und an beiden Seiten jeweils eine niedrigschmelzende Deckschicht vorgesehen ist, so kann dann durch das Erstarren der Kernschicht nach der Extrusion eine ausreichende Stabilität der Polymerschmelze, der daraus resultierenden elastischen Folie und des Laminates erreicht werden, während dann die niedrigschmelzenden Deckschichten zunächst noch eine Verbindung mit den vorzugsweise aus Nonwoven gebildeten Decklagen ermöglichen. Entsprechende Deckschichten können gegebenenfalls auch leichter wieder angeschmolzen werden,

Wenn gemäß der beschriebenen bevorzugten Ausgestaltung der Erfindung zwei Decklagen in aufeinanderfolgenden Schritten mit der Extrusionsbahn der elatischen Folie kaschiert werden, so kann dann vor der Kaschierung der zweiten Deckschicht gegebenenfalls auch die entsprechende Seite der Extrusionsbahn bzw. der elastischen Folie auch wieder auf eine gewünschte Temperatur aufgeheizt werden, wozu beispielsweise Heißluft oder Infrarotstrahlung in Betracht kommen.

Wie bereits eingangs beschrieben, wird die erste Decklage und gegebenenfalls die zweite Decklage in gleicher Weise mit der zugeordneten Dehneinrichtung gedehnt und dabei gleichzeitig in Falten gelegt. Dabei ist es möglich, dass sich die Decklage bzw. die Decklagen vor und nach dem Dehnen über die gleiche Breite erstrecken, auch wenn die Decklage bzw. die Decklagen durch die Wellenform nach dem Dehnen an sich eine größere Breite aufweisen.

So kann die erste Decklage und gegebenenfalls auch die zweite Decklage der jeweils zugeordneten Dehneinrichtung mit einer Breite zugeführt werden, welche in etwa der Breite und vorzugsweise genau der Breite der Extrusionsbahn bzw. der elastischen Folie bei der Kaschierung entspricht.

Es ist auch zweckmäßig, wenn die erste Materialbahn und gegebenenfalls die zweite Materialbahn in der zugeordneten Dehneinrichtung an ihren Rändern festgehalten wird, um die quer zur Produktionsrichtung von der Materialbahn abgedeckte Breite konstant zu halten. Die für die Bereitstellung einer Dehnbarkeit des Laminates gewünschte Verlängerung der Decklage selbst erfolgt dann dadurch, dass die entsprechende Decklage gedehnt und wellenförmig in Falten gelegt wird.

Gemäß einer weiteren Variante kann ein elastisches Laminat mit zwei einander gegenüberliegenden Decklagen auch dadurch gebildet werden, dass zwei Abschnitte des zuvor beschriebenen elastischen Laminates mit der elastischen Folie und lediglich der ersten Decklage an der elastischen Folie miteinander verbunden werden. Zur besseren Unterscheidung wird eine solche Ausgestaltung im Rahmen der Erfindung auch als Doppellaminat bezeichnet.

Das Doppellaminat kann dadurch gebildet werden, dass zwei Bahnen des Laminates mit der elastischen Folie und lediglich der ersten Deckschicht zugeführt werden, wobei die jeweils die elastische Folie aufweisenden Seiten einander zugewandt sind. Sodann erfolgt dort eine Verbindung der elastischen Folien. Die beiden zugeführten Laminate können grundsätzlich unterschiedlich oder gleich ausgeführt sein. Beispielsweise können je nach Anwendungszweck unterschiedliche Deckschichten vorgesehen sein. Bevorzugt sind die elastischen Folien der beiden als Bahn zugeführten Laminate aus dem gleichen Material gebildet, so dass diese gut miteinander verbunden werden können und des Weiteren bei dem Doppellaminat einen weitgehend einheitlichen elastischen Kern bilden. Eine Verbindung ist dabei je nach eingesetztem Material auf unterschiedliche Weise möglich. Wenn die elastische Folie aufgrund ihrer elastischen Eigenschaften auch vergleichsweise weich und klebrig ist, kann unter Umständen eine ausreichende Verbindung alleine durch Druck, beispielsweise ein Zusammendrücken in einem Walzenspalt ausreichend sein. Die einander zugewandten elastischen Folien können für eine Kaschierung vorzugsweise zumindest oberflächlich erwärmt werden, um dann mit einfachen Mitteln eine feste und zuverlässige Verbindung zu erreichen. Eine entsprechende Erwärmung ist beispielsweise mit Infrarotstrahlung und/oder Heißluft möglich.

Das zuvor beschriebene Doppellaminat kann des Weiteren auch dadurch gebildet werden, dass eine Bahn des Laminates umfassend die elastischen Folie und lediglich die erste Deckschicht an der Seite der elastischen Folie, insbesondere entlang einer in Produktionsrichtung verlaufenden Faltkante, auf sich selbst gefaltet wird. Wenn dies unmittelbar nach der Verbindung der Extrusionsbahn der ersten Deckschicht erfolgt, ist die elastische Folie bzw. die Extrusionsbahn unter Umständen noch so warm und klebrig, dass eine Verbindung der im gefalteten Zustand doppellagigen elastischen Folie mit sich selbst ohne weitere Erwärmung möglich ist. Selbstverständlich kann aber im Rahmen der beschriebenen Ausführungsform eine Erwärmung mittels Infrarotstrahlung und/oder Heißluft vorgesehen sein.

In diesem Zusammenhang ist grundsätzlich darauf hinzuweisen, dass die Bildung des Doppellaminates aus zwei Abschnitten des Laminates vollständig separat (offline) zu der Bildung des Laminates an sich erfolgen kann. Das Laminat kann in einem separaten und abgeschlossenen Prozess gebildet und gegebenenfalls ausgelagert werden. Eine Bahn des Laminates kann dann für die Bildung des Doppellaminates gefaltet oder in zwei Bahnen halber Breite geschnitten werden. Des Weiteren können für die Bildung des Doppellaminates auch zwei Bahnen des zuvor gefertigten Laminates bereitgestellt werden, so dass dann kein Falten oder Schneiden notwendig ist.

Erfindungsgemäß weist das Laminat zumindest eine erste Decklage auf. Davon ausgehend wurden zuvor auch Varianten mit zwei Decklagen beschrieben, welche in Falten gelegt sind und so mit der Extrusionsbahn der elastischen Folie verbunden werden. Grundsätzlich sich aber auch weitere Varianten möglich, bei welchen die erste Decklage wie beschrieben in Falten gelegt ist, eine zweite Decklage jedoch eine andere Ausgestaltung aufweist. Beispielsweise kann als zweite Decklage auch ein nur schwach gebundenes, leicht dehnbares Material, insbesondere ein Nonwoven vorgesehen sein. Ein solches leicht dehnbares Material muss dann nicht in Falten gelegt werden, um eine ausreichende Dehnbarkeit des gesamten Laminates zu ermöglichen. Die mechanischen Eigenschaften Laminates wie die für eine Dehnung notwendige Kraft, die elastischen Rückstellkräfte sowie eine wahrnehmbare Dehngrenze werden dann im Wesentlichen von der von der elastischen Folie und der in Falten gelegten ersten Decklage bereitgestellt bzw. bestimmt. Bei der Herstellung der beschriebenen Variante kann vorzugsweise vorgesehen sein, dass zunächst die zweite Decklage flächig mit der Extrusionsbahn verbunden wird, während diese nach dem Austritt aus einem Düsenspalt noch weitgehend schmelzflüssig und somit sehr klebrig ist. Es kann dann auch bei einem vergleichsweise geringen flächigen oder abschnittsweisen Andruck eine verlässliche Verbindung erreicht werden. Die in Falten gelegte erste Decklage wird nachfolgend mit der Extrusionsbahn verbunden, wobei dann durch die Verbindung lediglich an linienförmigen Abschnitten ohne weiteres lokal relativ große Andruckkräfte vorgesehen sein können.

Die erste Materialbahn und gegebenenfalls auch die zweite Materialbahn kann mit der zugeordneten Dehneinrichtung beispielsweise in Bezug auf eine Ausgangslänge um einen Wert zwischen 100 % und 400 %, insbesondere zwischen 150 % und 350 % gedehnt werden. Wenn die Materialbahn bzw. die Materialbahnen dann mit einer entsprechenden Wellenform kaschiert werden, entspricht dieser Wert auch in etwa der Dehnbarkeit des Laminates, bis die zunächst wellenförmig auf der elastischen Folie aufliegende Decklage wieder im Wesentlichen gerade gezogen ist, wobei dann üblicherweise für einen Benutzer eine Dehngrenze deutlich wahrnehmbar ist.

In diesem Zusammenhang ergibt sich auch ein weiterer Vorteil der vorliegenden Erfindung. Einerseits kann durch die erfindungsgemäß vorgesehene Dehnung zumindest der ersten Decklage das Material, also insbesondere Nonwoven ausgedünnt werden, wodurch (in Bezug auf den gedehnten Zustand des Laminates) das Flächengewicht der Decklage reduziert wird. Andererseits erfolgt die Dehnung in der Dehneinrichtung auch so, dass die zugeordnete, vorzugsweise von einem Nonwoven gebildete Decklage nicht in ihrer Struktur zerstört wird.

Wie sich die Dehnung der Decklage bzw. der Decklagen im Einzelnen auswirken, hängt nicht nur von dem Grad der Dehnung sondern wesentlich auch von dem Material der Decklage ab, wobei unterschiedliche Typen von Nonwoven auch unterschiedliche Eigenschaften aufweisen können.

So sind Nonwoven-Materialien verfügbar, bei denen bei einem Kraft-Dehnungsdiagramm zunächst eine relativ leichte Dehnbarkeit beobachtet wird, wobei die Faserstruktur nach Art eines Netzes auseinandergezogen wird. Das Material wird dann ausgedünnt, jedoch in seiner eigentlichen Struktur nicht zerstört. Teils wird in dem Kraft-Dehnungsdiagramm dann mit zunehmender Dehnung ein relativ abrupt einsetzender Anstieg der Kraft beobachtet, wobei dieser Bereich der Kurve auch als Knie bezeichnet werden kann. Für eine weitere Dehnung sind dann deutlich größere Kräfte notwendig, die auch mit einer Zerstörung des Materials einhergehen kann. Bevor dann schließlich das Nonwoven zerreißt, fällt die für eine weitere Dehnung notwendige Kraft üblicherweise wieder deutlich ab.

Die zuvor beschriebene Charakteristik mit der Ausbildung eines Knies lässt sich auch dadurch charakterisieren, dass das Kraft-Dehnungsdiagramm einen Wendepunkt aufweist. So ist gemäß einer bevorzugten Ausgestaltung der Erfindung vorgesehen, dass ein Nonwoven mit einem Kraft-Dehnungsdiagramm eingesetzt wird, bei dem in einem Bereich zwischen 100 % Dehnung und 300 % Dehnung ein Wendepunkt auftritt. Die Reißdehnung des Nonwovens beträgt üblicherweise zumindest 300 %, vorzugsweise zumindest 400 % und insbesondere zumindest 500 %.

Unter Berücksichtigung der zuvor beschriebenen Charakteristik kann die zunächst leichte Dehnung ohne Weiteres dazu vorgesehen werden, um das Material auszudünnen und somit ein besonders weiches und kostengünstiges Laminat zu fertigen. Wenn dann im Rahmen der Erfindung die Dehnung bis in etwa zu dem zuvor beschriebenen Wendepunkt oder Knie durchgeführt wird, bleibt der dann in dem Kraft-Dehnungsdiagramm anschließende steile Anstieg der Kraft bei einer zusätzlichen Dehnung erhalten und wirkt bei dem so gebildeten Laminat als deutlich wahrnehmbare Dehngrenze.

Die beschriebene Charakteristik tritt insbesondere bei verfestigten Nonwoven-Materialien auf, wobei gemäß einer ersten bevorzugten Ausgestaltung der Erfindung das Nonwoven zumindest eine Spunlace-Schicht aufweist. Als Spunlace-Nonwoven wird ein Nonwoven bezeichnet, welches durch Wasserstrahlen verfestigt bzw. vernadelt ist.

Zusätzlich oder alternativ kann des Nonwoven auch zumindest eine Spunbond-Schicht aufweisen. Besonders bevorzugt ist in diesem Zusammenhang eine Ausgestaltung, bei der die entsprechende Spunbond-Schicht gekräuselte Fasern aufweist oder vollständig aus gekräuselten Fasern besteht. Die Kräuselung kann beispielsweise auf eine mechanische Kräuselung und/oder den Einsatz von Bikomponenten-Fasern mit einer exzentrischen Materialstruktur zurückzuführen sein. Wenn beispielsweise bei Bikomponenten-Fasern Polymermaterialien mit unterschiedlichen Schrumpfverhalten nebeneinander angeordnet werden (side-by-side-Anordnung), kann sich dann eine Kräuselung bereits bei dem Abkühlen oder durch eine zusätzliche Wärmebehandlung ergeben.

Die Kräuselung der Fasern kann im Rahmen der Erfindung aus verschiedenen Gründen vorteilhaft sein. So sind entsprechende Spunbond-Schichten wegen der Kräuselung der Fasern für einen Benutzer angenehm weich. Vor diesem Hintergrund werden solche Nonwoven-Materialen in der Praxis auch als High-Loft-Nonwoven bezeichnet. Die hohe Weichheit kommt besonders dann zum Tragen, wenn die Spunbond-Schicht mit gekräuselten Fasern eine Oberfläche des Laminates bildet. Entsprechende Nonwoven und deren Vorteile sind beispielsweise in der EP 3 246 444 A1 beschrieben.

Spunbond-Schichten aus gekräuselten Fasern zeichnen sich auch durch eine gute Dehnbarkeit aus. Bei einer Dehnung können dabei die gekräuselten Fasern leicht zunächst in einem gewissen Maße entlang der Dehnungsrichtung gerade gezogen werden, ohne dass die Struktur der entsprechenden Nonwoven-Schicht zerstört wird. Wenn bei einem Nonwoven oder einer Schicht des Nonwoven nur ein Anteil der Fasern wie beschrieben gekräuselt ist, kann zumindest dieser Anteil in einem gewissen Maße zerstörungsfrei gedehnt werden.

Grundsätzlich kommen aber auch andere Nonwoven-Materialien in Betracht, wobei insbesondere auch ein mehrschichtiges Nonwoven eingesetzt werden kann. Die mechanischen sowie die haptischen Eigenschaften des Nonwovens können gerade bei einem mehrschichtigen Aufbau genau eingestellt und ausgewählt werden.

Grundsätzlich ist es auch möglich, dass das gesamte Nonwoven, eine Schicht des Nonwoven oder zumindest ein Teil der Fasern einer Schicht aus nichtschmelzenden Fasern wie beispielsweise Viskose, Baumwolle oder Cellulose gebildet ist. Nichtschmelzende Fasern können beispielsweise vorgesehen sein, um den Anteil nachwachsender Rohstoffe oder den Laminat spezifische Eigenschaften wie eine gewisse Saugfähigkeit zu verleihen.

Wenn im Rahmen der Erfindung eine Thermobondierung durch Druck und Temperatur erfolgt, kann auf einen zusätzlichen Klebstoff verzichtet werden. Neben einer entsprechenden Kostenersparnis werden auch andere zum Teil mit Klebstoffen verbundene Nacheile, wie beispielsweise die Freisetzung flüchtiger Komponenten vermieden.

Wie bereits zuvor beschrieben, kann die Extrusionsbahn bzw. die elastische Folie mehrschichtig aufgebaut sein. So kann beispielsweise bei einem dreischichtigen Aufbau eine Kernschicht aus thermoplastischen Elastomer mit nicht-elastischen Deckschichten kombiniert werden.

Als thermoplastisches Elastomer kommen bei einer ein- oder mehrschichtigen Ausgestaltung der Extrusionsbahn bzw. der elastischen Folie übliche Styrol-Block-Copolymere, wie auch thermoplastische Elastomere auf der Basis von Polyolefin (TPE-O) in Betracht, wobei diese häufig von einem Polypropylen-Copolymer gebildet sind. Bei einem mehrschichtigen Aufbau ist dabei zumindest eine elastische Schicht vorzusehen. Beispielsweise kann eine Kernschicht aus elastischen Polyolefin zwischen dünnen Deckschichten angeordnet sein, die ebenfalls aus Polyolefin gebildet sind. Insbesondere bei vergleichsweise dünnen Decksichten ist es dann nicht notwendig, dass die Deckschichten aus einem elastischen Polyolefin gebildet sind.

Gegenstand der Erfindung ist auch ein Laminat, welches durch das zuvor beschriebene Verfahren erhältlich ist.

Das Laminat weist eine erste Decklage aus Nonwoven und eine elastische Folie auf, wobei die erste Decklage und die elastische Folie lediglich an entlang einer Längsrichtung verlaufenden durchgehenden oder unterbrochenen Abschnitten durch eine Thermobondierung mit der Folie verbunden sind, wobei die erste Decklage entlang einer Querrichtung wellenförmig mit Bögen zwischen den verbundenen Abschnitten auf der Folie angeordnet ist und wobei die erste Decklage unter Einbeziehung der Wellenform mit den freiliegenden Bögen entlang der Querrichtung zumindest die doppelte Breite der Folie aufweist und wobei die Folie nach ihrer Bildung des Laminates durch die Thermobondierung und vorzugweise auch ausgehend von der Herstellung der Folie selbst ungedehnt ist. Üblicherweise liegen die Bögen zwischen den verbundenen Abschnitten frei. Gerade bei einer vergleichsweise geringen Dehnung und somit weniger stark ausgeprägten Wellenform ist nicht ausgeschlossen, dass an den Bögen zumindest einzelne Fasern eines Nonwovens als bevorzugte Decklage leicht mit der Folie verbunden sind.

Vorzugsweise ist vorgesehen, dass die Decklage bezogen auf die Grundfläche des Laminates im ungedehnten Zustand ein Flächengewicht zwischen 10 g/m² und 80 g/m² aufweist. Wenn wie zuvor im Zusammenhang mit dem Verfahren beschrieben, die Decklage bei der Dehnung an ihren Rändern festgehalten wird und die in Querrichtung abgedeckte Fläche unverändert bleibt, entspricht das angegebene Flächengewicht auch dem anfänglich bei der entsprechenden Materialbahn vorgesehenen Flächengewicht. Bezogen auf den gedehnten Zustand des Laminates oder unter Berücksichtigung der tatsächlichen Länge der Deckschicht ergibt sich ein deutlich geringeres Flächengewicht. Dies führt auch gerade dazu, dass im Rahmen der Erfindung eine besonders effiziente Materialausnutzung erreicht wird.

Wie sich auch aus dem Verfahren ergibt, ist die erste Decklage durch das Verstrecken in Wellenform gebracht. Weitere Ausgestaltungsmöglichkeiten des Laminates selbst ergeben sich auch aus der Beschreibung des Verfahrens.

Die Erfindung wird im Folgenden einer anhand lediglich ein Ausführungsbeispiel darstellenden Zeichnung erläutert. Es zeigen:
- Fig. 1: Wesentliche Schritte eines Verfahrens zur Herstellung eines elastischen Laminates,
- Fig. 2: der Verfahrensschritt des Dehnens einer Decklage,
- Fig. 3a: eine schematische Ansicht eines elastischen Laminates an einem Querschnitt,
- Fig. 3b: das Laminat gemäß der Fig. 3a im gedehnten Zustand,
- Fig. 4: ein Querschnitt gemäß der Fig. 3a für eine alternative Ausgestaltung des elastischen Laminates,
- Fig. 5: ein Kraft-Dehnungsdiagramm für eine Decklage des Laminates,
- Fig. 6: eine Weiterbildung des Verfahrens gemäß der Fig. 1,
- Fig. 7: die Bildung eines Doppellaminates.

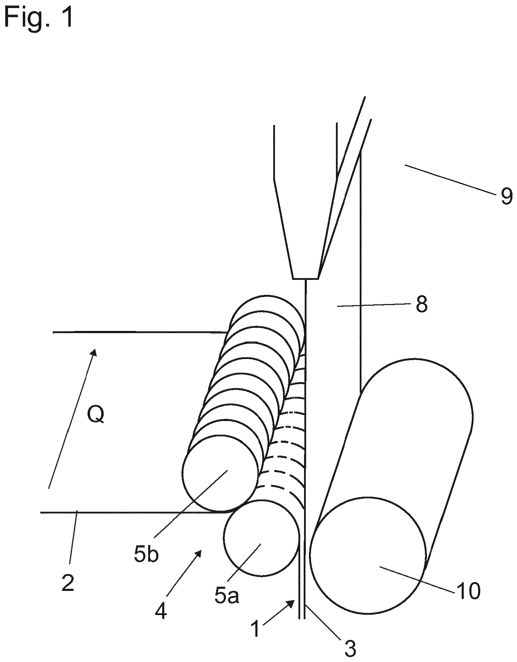

Die Fig. 1 zeigt ein Verfahren zur Herstellung eines elastischen Laminates 1 mit einer ersten Decklage 2 und einer elastischen Folie 3. Als erste Decklage 2 ist ein Spunlace-Nonwoven vorgesehen. Die erste Decklage 2 wird entlang einer Produktionsrichtung einer Dehneinrichtung 4 zugeführt. Als Produktionsrichtung wird dabei im Rahmen der Erfindung die Vortriebsrichtung der Materialbahn der ersten Decklage 2 bezeichnet, wobei sich die Produktionsrichtung bei einer Umlenkung an Walzen selbstverständlich entsprechend ändert. Die Zuordnung der Produktionsrichtung dient zur Abgrenzung und Definition einer Querrichtung Q. Wenn also mehrere Abschnitte oder Materialbahnen als Endlosbahn zugeführt werden, so müssen hierzu nicht die unterschiedlichen Zuführvorrichtungen unterschieden werden.

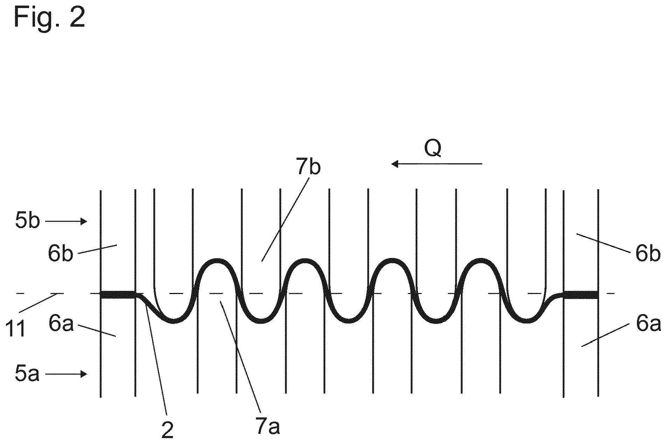

Die erste Dehneinrichtung 4 weist zwei ineinandergreifende Ringrollen 5a, 5b auf, die mit der dazwischen angeordneten Decklage 2 in der Fig. 2 in einem Querschnitt dargestellt sind. Aus einer vergleichenden Betrachtung der Figuren 1 und 2 ist zu erkennen, dass die Materialbahn der ersten Decklage 2 an Rändern 6a, 6b der Ringrollen 5a, 5b festgehalten ist, wobei zwischen den Rändern 6a, 6b die Ringrollen 5a, 5b jeweils gegenüber einer Mittelebene M vorstehende Ringe 7a, 7b in Form von Zähnen vorgesehen sind. Da die Materialbahn der ersten Decklage 2 an den Rändern 6a, 6b festgehalten wird, ändert sich auch in der ersten Dehneinrichtung 4 nicht die in Querrichtung Q von der ersten Decklage 2 abgedeckte Breite. Die ineinandergreifenden Ringe 7a, 7b bewirken jedoch eine Verlängerung, d. h. Dehnung der Materialbahn der ersten Decklage 2. Der Grad der Dehnung ergibt sich aus der Form der Ringe 7a, 7b sowie aus dem Versatz gegenüber der Mittelebene M.

Die Dehnung beträgt üblicherweise zwischen 50 % und 400 %, insbesondere zwischen 100 % und 350 %, bevorzugt zwischen 150 % und 300 %.

Die erste Decklage 2 wird an der ersten Dehneinrichtung 4 so geführt, dass auch nach Verlassen des Walzenspaltes die erste Decklage 2 an einer der Ringrollen 5a weiterhin anliegt und geführt ist. Die erste Decklage behält so also auch nach dem Verlassen des Walzenspaltes im Wesentlichen die in Fig. 2 dargestellte Struktur.

Während die erste Decklage dann an der zugeordneten Ringrolle 5a geführt ist, erfolgt eine Kaschierung der ersten Decklage 2 mit einer Extrusionsbahn 8, die aus einer Cast-Düse 9 austritt. Die Kaschierung der ersten Decklage 2 mit der Extrusionsbahn 8 erfolgt zwischen einem Walzenspalt der zugeordneten Ringrolle 5a und einer Gegenwalze 10, welche die Extrusionsbahn 8 abkühlt und somit auch glättet und fixiert. Die Gegenwalze 10 kann deshalb auch als Glättwalze oder Chill-Walze bezeichnet werden.

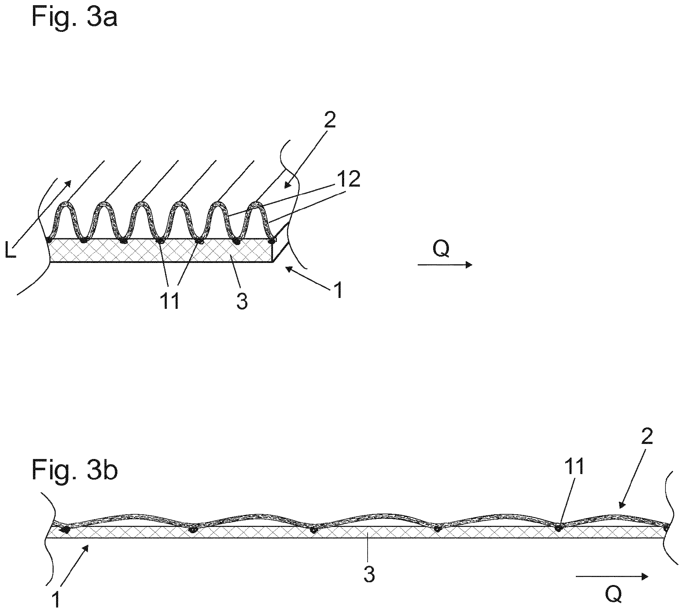

Da die erste Decklage 2 bei der Kaschierung mit der Extrusionsbahn 8 an einer der Ringrollen 5a geführt ist, werden die erste Decklage 2 und die Extrusionsbahn 8 nur an linienförmigen Abschnitten 11 verbunden, während zwischen den Abschnitten 11 die erste Decklage 2 im Wesentlichen auch die in der Fig. 2 dargestellte Wellenform mit Falten beibehält. Zwischen den verbundenen Abschnitten 11 verbleiben freiliegende Bögen 12, welche zu einer besonders weichen und angenehmen Haptik beitragen (siehe Fig. 3a).

Gemäß der Fig. 3a sind die Abschnitte 11 in Produktionsrichtung und damit in einer Längsrichtung L des Laminates 1 durchgehend. Grundsätzlich ist es aber auch möglich, dass die Abschnitte 11 entlang der Längsrichtung L unterbrochen sind. Dies kann dadurch erreicht werden, dass die einzelnen Ringe 7a, 7b entlang der Umfangsrichtung der zugeordneten Ringrollen 5a, 5b unterbrochen, d. h. segmentiert sind.

Gemäß der Fig. 3b ist das Laminat 1 so weit gedehnt, dass die in Fig. 3a dargestellten Bögen 12 auseinandergezogen sind, sodass dann die erste Decklage 2 weitgehend flach auf der elastischen Folie 3 aufliegt. Die Breite des Laminates entlang der Querrichtung Q entspricht dann der Gesamtbreite der ersten Decklage unter Berücksichtigung der Wellenform. Durch den in Fig. 2 dargestellten Grad der Dehnung in der ersten Dehnrichtung 4 wird somit auch für das gesamte Laminat 1 eine Dehngrenze vorgegeben. Bis zum Erreichen der in Fig. 3b dargestellten Konfiguration ist das Laminat relativ leicht dehnbar, wobei für die elastischen Eigenschaften und die für eine Dehnung notwendigen Kräfte im Wesentlichen die elastische Folie 3 ausschlaggebend ist.

Bei Erreichen der in Fig. 3b dargestellten Konfiguration ist dann die erste Decklage 2 stramm gezogen und bewirkt eine deutlich wahrnehmbare Dehngrenze, die von einem Benutzer als eine Art Anschlag wahrgenommen wird. Durch diese Maßnahmen kann verhindert werden, dass das Laminat 1 bei der Benutzung versehentlich überdehnt und somit unter Umständen zerstört wird.

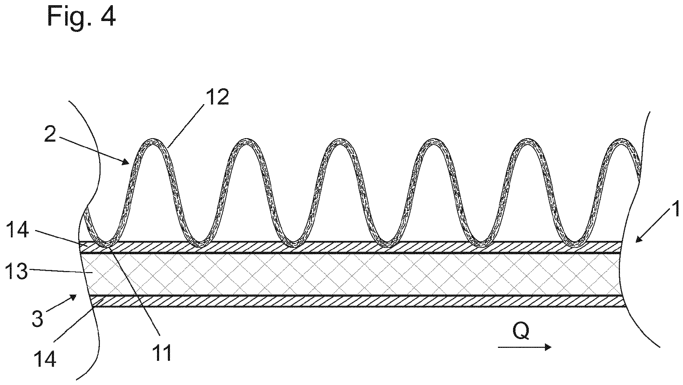

Die Extrusionsbahn 8 bzw. die daraus gebildete elastische Folie 3 kann ohne Einschränkung ein- oder mehrschichtig sein. Die Fig. 4 zeigt in diesem Zusammenhang exemplarisch einen dreischichtigen Aufbau der elastischen Folie 3, wobei eine Kernschicht 13 aus thermoplastischen Elastomer zwischen Deckschichten 14 angeordnet ist, die vorzugsweise nicht oder weniger elastisch sind. Zumindest ist gerade bei dünnen Deckschichten 14 eine Elastizität für die grundlegende Funktion des Laminates nicht notwendig. Dabei kann auch vorgesehen sein, dass die Deckschichten 14 aus einem niedrigschmelzenden Material sind, sodass nach dem Austritt aus der Cast-Düse 9 die Kernschicht 13 relativ schnell eine geschlossene homogene Schicht bildet, während die Deckschichten 14 noch für eine gute Kaschierung der zumindest ersten Decklage 2 zur Verfügung stehen. Dabei kann auch vorgesehen sein, dass die vorzugsweise aus Nonwoven gebildete erste Decklage 2 durch die Kaschierung in die Extrusionsbahn 8 und insbesondere die Deckschichten 14 eindringt. An den verbundenen Abschnitten 11 ist das Nonwoven somit besonders zuverlässig in die elastische Folie 3 eingebettet und kann dann lediglich durch eine Zerstörung wieder getrennt werden.

Zusätzlich oder alternativ kann auch vorgesehen sein, dass die aus Nonwoven gebildeten Decklagen unterschiedlich schmelzenden Fasern und/oder Bikomponenten-Fasern aufweist. Bei Bikomponenten-Fasern kann beispielsweise ein niedrigschmelzender Mantel um einen hochschmelzenden Kern vorgesehen sein, sodass dann durch ein Aufschmelzen des Mantels bei der Verbindung mit der Extrusionsbahn 8 ebenfalls ein Beitrag zu einer besonders hohen Verbundfestigkeit geleistet wird. Bei einem Fasergemisch kann ein ähnlicher Effekt durch einen Anteil niedrigschmelzender Fasern erreicht werden. Im Rahmen der Erfindung können auch nichtschmelzende Fasern wie beispielweise Viskose oder Baumwolle vorgesehen sein, welche bei der Herstellung in die Extrusionsbahn hineingedrückt und somit mechanisch verbunden werden.

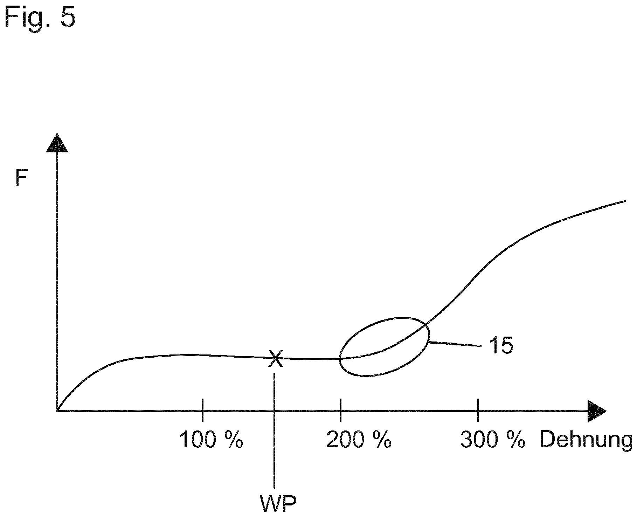

Wie bereits zuvor erläutert, ist beispielsweise ein Spunlace-Nonwoven als Decklage 2 im besonderen Maße geeignet. Hierzu wird gemäß der Fig. 5 exemplarisch ein typisches Kraft-Dehnungsdiagramm dargestellt, wobei das Nonwoven in einem Bereich zwischen 100 % Dehnung und 300 % Dehnung einen Wendepunkt WP aufweist. Anschließend an den Wendepunkt WP steigt dann die für eine weitere Dehnung notwendige Kraft F stark an, sodass das Kraft-Dehnungsdiagramm eine Art Knie 15 aufweist. Zweckmäßigerweise wird das Nonwoven bis in etwa zu dem Wendepunkt WP bzw. dem Knie 15 wie in Fig. 2 dargestellt vor der Kaschierung gedehnt, sodass das Material auf vorteilhafte Weise ausgedünnt wird, ohne seine Struktur zu verlieren. Der an das Knie 15 anschließende deutliche Kraftanstieg bleibt dann jedoch auch in Hinblick für das Laminat 1 noch erhalten, wodurch die zuvor beschriebene deutlich wahrnehmbare Dehngrenze sichergestellt wird. Die Reißdehnung des Nonwovens liegt typischerweise oberhalb von 100 %, vorzugsweise oberhalb von 150 % und insbesondere oberhalb von 200 %. Je nach Ausgestaltung des Nonwovens kann die Reißdehnung aber auch oberhalb von 300 %, 400 % oder auch 500 % liegen.

Gemäß einer Weiterbildung der Erfindung ist vorgesehen, dass das Laminat gegenüberliegend der ersten Decklage 2 auch eine zweite Decklage 16 aufweist, die wie zuvor im Zusammenhang mit der ersten Decklage 2 beschrieben gebildet sein kann. Entsprechend wird also die zweite Decklage 16 einer zweiten Dehneinrichtung 17 mit Ringrollen 18a, 18b zugeführt wobei bezüglich der weiteren Details auf die Ausführungen zu der ersten Dehneinrichtung 4 verwiesen wird.

Die zweite Decklage 16 wird mit einem geringen Versatz mit der Extrusionsbahn 8 kaschiert. Dabei muss sichergestellt werden, dass die Extrusionsbahn 8 auch bei der Kaschierung mit der zweiten Decklage 16 noch nicht soweit erstarrt ist, dass eine Verbindung nicht mehr möglich ist. Hierzu kann mit Verweis auf die Fig. 4 auch von Vorteil sein, wenn eine Kernschicht 13 aus thermoplastischen Elastomer mit Deckschichten 14 kombiniert wird, welche niedrigerschmelzend sind und somit auch bei einer Temperaturabnahme flüssig oder zumindest weich bleiben.

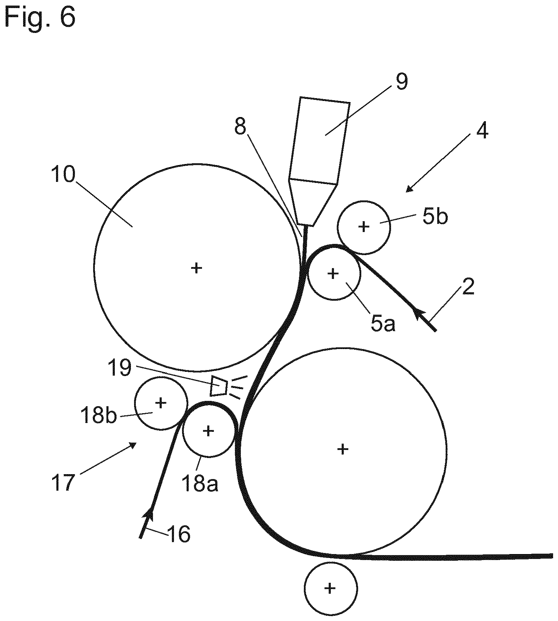

Grundsätzlich ist es auch möglich, vor der Kaschierung der Extrusionsbahn 8 mit der zweiten Decklage 16 die entsprechende Oberfläche der Extrusionsbahn 8 wieder in einem gewissen Maße aufzuwärmen, wozu in der Fig. 6 exemplarisch eine Heizeinrichtung 19 dargestellt ist. Die Heizeinrichtung 19 kann beispielsweise eine Erwärmung mittels Heißluft und/oder Infrarotstrahlung vorsehen.

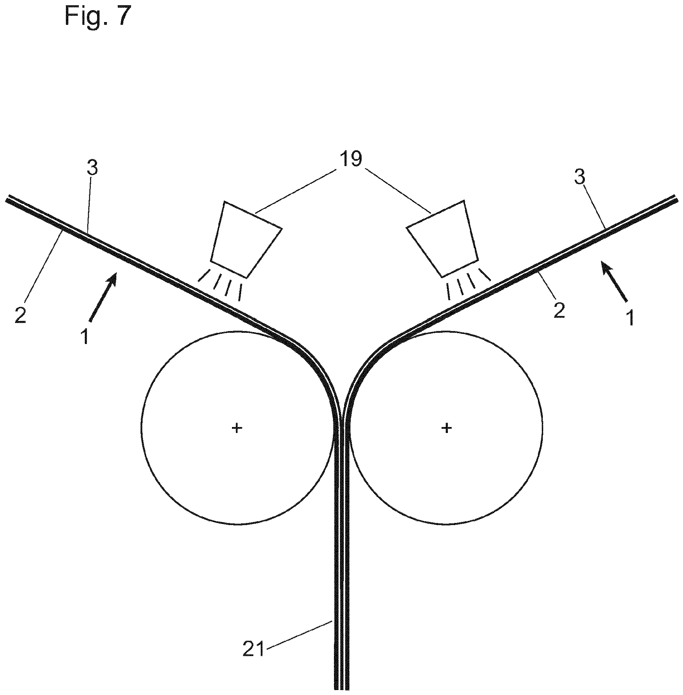

Durch die in der Fig. 6 dargestellte Ausführungsform ist es möglich, beidseitig der Extrusionsbahn 8 die erste Decklage 2 und die zweite Decklage 16 anzuordnen. Die Fig. 7 zeigt vor diesem Hintergrund eine alternative Ausgestaltung, wobei zwei bereits zuvor gebildete Laminate 1 mit jeweils lediglich der elastischen Folie 3 und der ersten Decklage 2 so zugeführt werden, dass die elastischen Folien 3 einander zugewandt sind. Nach einer zumindest oberflächlichen Erwärmung mittels Heizeinrichtungen 19 erfolgt eine Verbindung in einem Walzenspalt. Die beiden aufeinander liegenden elastischen Folien 3 verblocken dadurch zu einem weitgehend einheitlichen elastischen Kern 20. Es wird somit aus zwei Abschnitten des Laminates 1 eine Art Doppellaminat 21 gebildet.

## Patentansprüche

1. Verfahren zur Herstellung eines elastischen Laminates (1) mit zumindest einer ersten Decklage (2) und einer elastischen Folie (3), wobei
a) die erste Decklage (2) als erste Materialbahn entlang einer Produktionsrichtung einer ersten Dehneinrichtung (4) zugeführt wird,
b) die erste Decklage (2) nachfolgend mit der ersten Dehneinrichtung (4) quer zur Produktionsrichtung gedehnt und dabei in Falten gelegt wird und entweder
c) die in Falten gelegte erste Decklage (2) nachfolgend mit einer für die Bildung der elastischen Folien (3) vorgesehenen Extrusionsbahn (8) derart verbunden wird, dass die Decklage (2) an ihrer der Extrusionsbahn (8) zugewandten Seite lediglich an Abschnitten (11) mit der Extrusionsbahn (8) verbunden wird,
oder
d) die in Falten gelegte erste Decklage (2) nachfolgend mit eine vorgefertigten elastischen Folie (3) dadurch verbunden wird, dass die elastische Folie (3) an ihrer der ersten Decklage (2) zugewandten Seite zumindest angeschmolzen wird, dass die erste Decklage (2) lediglich an Abschnitten (11) gegen die zumindest angeschmolzene Seite der elastischen Folie (3) gedrückt wird und dass dabei die erste Decklage (2) zumindest teilweise in eine Polymermatrix der elastischen Folie (3) eingebettet wird.

2. Verfahren nach Anspruch 1, wobei die erste Dehneinrichtung (4) von zwei ineinandergreifenden Ringrollen (5a, 5b) gebildet ist.

3. Verfahren nach Anspruch 2, wobei die erste Materialbahn und die Extrusionsbahn (8) bzw. die elastische Folie (3) in einem Walzenspalt zwischen einer der Ringrollen (5a) und einer Gegenwalze (10) verbunden werden.

4. Verfahren nach einem der Ansprüche 1 bis 3, wobei eine zweite Decklage (16) als zweite Materialbahn einer zweiten Dehneinrichtung (17) zugeführt wird, mittels der zweiten Dehneinrichtung (17) quer zur Produktionsrichtung gedehnt und dabei in Falten gelegt wird und wobei die zweite Decklage (16) nachfolgend in Falten gelegt mit der Extrusionsbahn (8) bzw. der elastischen Folie (3) verbunden wird.

5. Verfahren nach einem der Ansprüche 1 bis 4, wobei die erste Materialbahn mittels der ersten Dehneinrichtung (4) um einen Wert zwischen 100 % und 400 % gedehnt wird.

6. Verfahren nach einem der Ansprüche 1 bis 5, wobei als erste Materialbahn ein Nonwoven zugeführt wird.

7. Verfahren nach Anspruch 6, wobei das Nonwoven zumindest eine Spunlace-Schicht aufweist.

8. Verfahren nach Anspruch 6 oder 7, **dadurch gekennzeichnet, dass** das Nonwoven zumindest eine Spunbond-Schicht aufweist.

9. Verfahren nach Anspruch 8, **dadurch gekennzeichnet, dass** die Spunbond-Schicht gekräuselte Fasern enthält und insbesondere aus gekräuselten Fasern gebildet ist.

10. Verfahren nach einem der Ansprüche 6 bis 9 wobei das Nonwoven eine Reißdehnung von mindestens 300 % aufweist und wobei ein Kraft-Dehnungsdiagramm des Nonwoven in einem Bereich zwischen 100 % Dehnung und 300 % Dehnung einen Wendepunkt (WP) aufweist.

11. Verfahren nach einem der Ansprüche 6 bis 10, wobei das Nonwoven Bikomponenten-Fasern enthält.

12. Verfahren nach einem der Ansprüche 1 bis 11, wobei die Extrusionsbahn (8) bzw. die elastische Folie (3) mehrschichtig mit einer Schicht aus thermoplastischem Elastomer und zumindest einer Deckschicht (14) gebildet wird.

13. Verfahren nach einem der Ansprüche 1 bis 12, wobei die Extrusionsbahn (8) die elastische Folie (3) aus Polyolefin gebildet ist.

14. Verfahren nach einem der Ansprüche 1 bis 13, wobei die erste Decklage (2) der ersten Dehneinrichtung (4) mit einer Breite zugeführt wird, welche zwischen 90 % und 110 % der Breite der Extrusionsbahn (8) bzw. der elastischen Folie (3) bei der Kaschierung entspricht.

15. Verfahren nach einem der Ansprüche 1 bis 14, wobei die erste Materialbahn in der ersten Dehneinrichtung (4) an ihren Rändern festgehalten wird.

16. Laminat erhältlich nach dem Verfahren nach einem der Ansprüche 1 bis 15 mit zumindest einer ersten Decklage (2) aus Nonwoven und einer elastischen Folie (3),
i) wobei die erste Decklage (2) und elastische Folie (3) lediglich an entlang einer Längsrichtung (L) verlaufenden Abschnitten (11) ohne einen separaten Klebstoff mit der Folie (3) verbunden ist,
ii) wobei die erste Decklage (2) entlang einer Querrichtung (Q) wellenförmig mit freiliegenden Bögen (12) zwischen den verbundenen Abschnitten (11) auf der Folie (3) angeordnet ist,
iii) wobei die erste Decklage (2) unter Einbeziehung der Wellenform mit den freiliegenden Bögen (12) entlang der Querrichtung (Q) zumindest die doppelte Breite der Folie (3) aufweist und
iv) wobei die Folie (3) nach ihrer Bildung ungedehnt ist.

17. Laminat nach Anspruch 16, wobei die erste Decklage (2) bezogen auf die Grundfläche des Laminates im ungedehnten Zustand ein Flächengewicht zwischen 15 g/m² und 80 g/m² aufweist.

18. Laminat nach Anspruch 16 oder 17, wobei die erste Decklage (2) durch ein Verstrecken in die Wellenform gebracht ist.

## Claims

1. Method for the production of an elastic laminate (1) with at least a first top layer (2) and an elastic film (3), wherein
a) the first top layer (2) is fed into a first stretching device (4) as the first material sheet along a direction of production,
b) the first top layer (2) is subsequently stretched using the first stretching device (4) transversely to the direction of production and thereby folded, and either
c) the first top layer (2) laid in folds is subsequently connected to an extrusion sheet (8) intended for the formation of the elastic films (3) in such a way that the top layer (2) is connected to the extrusion sheet (8) on its side facing the extrusion sheet (8) only at sections (11),
or
d) the folded first top layer (2) is subsequently connected to a prefabricated elastic film (3) by at least melting the elastic film (3) on its side facing the first top layer (2), that the first top layer (2) is pressed against the at least melted side of the elastic film (3) only at sections (11) and that the first top layer (2) is at least partially embedded in a polymer matrix of the elastic film (3).

2. The method according to Claim 1, wherein the first stretching device (4) is formed by two interlocking ring rollers (5a, 5b).

3. The method according to Claim 2, wherein the first material sheet and the extrusion sheet (8) or the elastic film (3) are connected in a roller gap between one of the ring rollers (5a) and a counter roller (10).

4. The method according to any one of the Claims 1 to 3, wherein a second top layer (16) is fed into a second stretching device (17) as a second material sheet and is stretched transversely to the direction of production by means of the second stretching device (17), thereby being folded, and wherein the second top layer (16) is subsequently folded and connected to the extrusion sheet (8) or the elastic film (3).

5. The method according to any one of the Claims 1 to 4, wherein the first material sheet is stretched by a value between 100 % and 400 % by means of the first stretching device (4).

6. The method according to any one of the Claims 1 to 5, wherein a nonwoven-material sheet is fed as the first material sheet.

7. The method according to claim 6, wherein the nonwoven material comprises at least one spunlace layer.

8. The method according to Claim 6 or 7, **characterized in that** the nonwoven material comprises at least one spunbond layer.

9. The method according to Claim **8, characterized in that** the spunbond layer contains crimped fibres and is made of crimped fibres in particular.

10. The method according to any one of the Claims 6 to **9,** wherein the nonwoven material comprises an elongation at break of at least 300 % and wherein a stress-strain diagram of the nonwoven comprises a inflection point (WP) at a range between 100 % elongation and 300 % elongation.

11. The method according to any one of the Claims 6 to 10, wherein the nonwoven material contains bicomponent fibres.

12. The method according to any one of the Claims 1 to 11, wherein the extrusion sheet (8) or the elastic film (3) is formed in multi-layer manner with a layer of thermoplastic elastomer and at least one surface layer (14).

13. The method according to any one of the Claims 1 to 12, wherein the extrusion sheet (8) or the elastic film (3) is made of polyolefin.

14. The method according to any one of the Claims 1 to 13, wherein the first top layer (2) is fed into the first stretching device (4) with a width equal to between 90 % and 110 % of the width of the extrusion sheet (8) or the elastic film (3) during lamination.

15. The method according to any one of the Claims 1 to 14, wherein the first sheet of material is held at its edges in the first stretching device (4).

16. A laminate available according to the method according to any one of the Claims 1 to 15 comprising at least a first top layer (2) of nonwoven material and an elastic film (3),
i) wherein the first top layer (2) and elastic film (3) are connected to the film (3) only at sections (11) running along a longitudinal direction (L) without a separate adhesive;
ii) wherein the first top layer (2) is arranged in an wave-like manner along a transverse direction (Q) with exposed arches (12) between the connected sections (11) on the film (3);
iii) wherein the first top layer (2), including the waveform with the exposed arches (12) along the transverse direction (Q), comprises at least twice the width of the film (3), and
iv) wherein the film (3) is unstretched after its formation.

17. The laminate according to Claim 16, wherein the first top layer (2) comprises a basis weight between 15 g/m² and 80 g/m² in relation to the base surface of the laminate in the unstretched state.

18. The laminate according to Claim 16 or 17, wherein the first top layer (2) is made into the wave shape by stretching it.

## Revendications

1. Procédé, destiné à fabriquer un stratifié (1) élastique, pourvu d'au moins une première couche de recouvrement (2) et d'un film (3) élastique,
a) la première couche de recouvrement (2) étant alimentée en tant que première bande de matière le long d'une direction de production d'un premier système d'allongement (4),
b) la première couche de recouvrement (2) étant ensuite allongée à l'aide du premier système d'allongement (4) à la transversale de la direction de production et posée en plis à cet effet et
c) la première couche de recouvrement (2) posée en plis étant assemblée ensuite avec une bande d'extrusion (8) prévue pour la création des films (3) élastiques, de telle sorte que sur sa face dirigée vers la bande d'extrusion (8), la couche de recouvrement (2) ne soit assemblée que sur des segments (11) avec la bande d'extrusion (8),
ou
d) la première couche de recouvrement (2) posée en plis étant assemblée ensuite avec un film (3) élastique préfabriqué en ce que sur sa face dirigée vers la première couche de recouvrement (2), le film (3) élastique est au moins mis en fusion, en ce que la première couche de recouvrement (2) est pressée uniquement sur des segments (11) contre la face mise en fusion du film (3) élastique et en ce qu'à cet effet, la première couche de recouvrement (2) est incorporée au moins partiellement dans une matrice en polymère du film (3) élastique.

2. Procédé selon la revendication 1, le premier système d'allongement (4) étant constitué de deux cylindres annulaires (5a, 5b) engrenés l'un dans l'autre.

3. Procédé selon la revendication 2, la première bande de matière et la bande d'extrusion (8) ou le film (3) élastique étant assemblés dans un espace pinceur entre l'un des cylindres annulaires (5a) et un contre-cylindre (10).

4. Procédé selon l'une quelconque des revendications 1 à 3, une deuxième couche de recouvrement (16) étant alimentée en tant que deuxième bande de matière vers un deuxième système d'allongement (17), étant allongée au moyen du système d'allongement (17) à la transversale de la direction de production et posée en plis à cet effet et posée en plis, la deuxième couche de recouvrement (16) étant assemblée ensuite avec la bande d'extrusion (8) ou avec le film (3) élastique.

5. Procédé selon l'une quelconque des revendications 1 à 4, la première bande de matière étant allongée au moyen du système d'allongement (4) d'une valeur comprise entre 100 % et 400 %.

6. Procédé selon l'une quelconque des revendications 1 à 5, en tant que première bande de matière étant alimenté un voile non-tissé.

7. Procédé selon la revendication 6, le voile non-tissé comportant au moins une couche de spunlace non tissé.

8. Procédé selon la revendication 6 ou 7, **caractérisé en ce que** le voile non-tissé comporte au moins une couche extrudée soufflée.

9. Procédé selon la revendication 8, **caractérisé en ce que** la couche extrudée soufflée contient des fibres frisées et est constituée notamment de fibres frisées.

10. Procédé selon l'une quelconque des revendications 6 à 9, le voile non-tissé faisant preuve d'une extension à la rupture d'au moins 300 % et un diagramme de force-allongement du voile non-tissé présentant un tournant (WP) dans une plage comprise entre un allongement à 100 % et un allongement à 300 %.

11. Procédé selon l'une quelconque des revendications 6 à 10, le voile non-tissé contenant des fibres à bicomposants.

12. Procédé selon l'une quelconque des revendications 1 à 11, la bande d'extrusion (8) ou le film (3) élastique étant constitué(e) en plusieurs couches avec une couche en élastomère thermoplastique et au moins une couche de couverture (14).

13. Procédé selon l'une quelconque des revendications 1 à 12, la bande d'extrusion (8) ou le film (3) étant constitué(e) de polyoléfine.

14. Procédé selon l'une quelconque des revendications 1 à 13, la première couche de recouvrement (2) étant alimentée vers le premier système d'allongement (4) avec une largeur qui correspond à entre 90 % et 110 % de la largeur de la bande d'extrusion (8) ou du film (3) élastique lors du pelliculage.

15. Procédé selon l'une quelconque des revendications 1 à 14, la première bande de matière étant maintenue sur ses bords dans le premier système d'allongement (4).

16. Stratifié, susceptible d'être obtenu d'après le procédé selon l'une quelconque des revendications 1 à 15, pourvu d'au moins une première couche de recouvrement (2) en voile non-tissé et d'un film (3) élastique,
i) la première couche de recouvrement (2) et le film (3) élastique étant assemblés avec le film (3) uniquement sur des segments (11) s'écoulant le long d'une direction longitudinale (L) sans agent adhésif séparé,
ii) la première couche de recouvrement (2) étant placée sur le film (3) le long d'une direction transversale (Q) sous forme ondulée avec des courbes (12) libres entre les segments (11) assemblés,
iii) la première couche de recouvrement (2) présentant sous prise en considération de la forme ondulée, avec les courbes (12) libres, le long de la direction transversale (Q) au moins le double de la largeur du film (3) et
iv) le film (3) étant non allongé après sa création.

17. Stratifié selon la revendication 16, à l'état non allongé, la première couche de recouvrement (2) présentant en rapport à la surface de base du stratifié un grammage compris entre 15 g/m² et 80 g/m².

18. Stratifié selon la revendication 16 ou 17, la première couche de recouvrement (2) étant amenée dans la forme ondulée par un étirement.
